# EUROPEAN PATENT SPECIFICATION

(11) **EP 0 812 842 B1**
(45) Date of publication and mention of the grant of the patent: **27.03.2002**
(21) Application number: 97303987.8
(22) Date of filing: 09.06.1997
(51) Int. Cl.: C07D 471/04, C07D 401/04, C07D 207/456

(54) **Process for the preparation of 5-(alkoxymethyl)-2,3-pyridine-dicarboximide compounds**
Verfahren zur Herstellung von 5-(Alkoxymethyl)-2,3-Pyridindicarbonsäureimidverbindungen
Procédé pour la préparation de 5-(alkoxyméthyl)-2,3-pyridinedicarboximides

(30) Priority: 10.06.1996 US 661289
(43) Date of publication of application: 17.12.1997
(73) Proprietor: BASF AKTIENGESELLSCHAFT, 67056 Ludwigshafen (DE)
(72) Inventor: Kremer, Kenneth Alfred Martin, Lawrenceville, New Jersey 08648 (US); Wu, Wen-Xue, Lawrenceville, New Jersey 08648 (US); Maulding, Donald Roy, Somerville, New Jersey 08876 (US)
(74) Representative: Köster, Reinhold, Dr.

(56) References cited:
- EP-A- 0 041 623
- EP-A- 0 172 140
- EP-A- 0 205 879
- EP-A- 0 308 084
- US-A- 4 353 734
- ADRIAN WALDNER: "[4+2]Cycloadditionen von alpha,beta-ungesättigten Hydrazonen" HELVETICA CHIMICA ACTA., vol. 71, no. 2, 16 March 1988, BASEL CH, pages 486-492, XP002055963

## Description

### BACKGROUND OF THE INVENTION

5 - (Alkoxymethyl)-2,3-pyridinedicarboximide compounds are useful as intermediates in the preparation of herbicidal 5-(alkoxymethyl)-2-(2-imidazolin-2-yl)nicotinic acid, ester and salt compounds. Methods for the preparation of 5-(alkoxymethyl)-2,3-pyridinedicarboximide compounds are rather limited, and those methods that are available may require extensive purification methods to provide high purity 5- (alkoxymethyl)-2,3-pyridinedicarboximide compounds.

European patent application number 308,084-A1 generically discloses that a 5-(alkoxymethyl)-2,3-pyridinedicarboximide compound may be prepared by reacting a substituted oxime compound with a halomaleimide compound optionally in the presence of an inorganic base.
However, that method is not satisfactory because it produces a mixture that contains the desired 5-(alkoxymethyl)-2,3-pyridinedicarboximide compound and a relatively high percentage of an undesirable 5-methyl-2,3-pyridinedicarboximide compound. Arduous or time consuming purification methods are then required to obtain high purity 5-(alkoxymethyl)-2,3-pyridinedicarboximide compounds. If the mixture is not highly purified, the 5-(alkoxymethyl)-2-(2-imidazolin-2-yl)nicotinic acid, ester or salt compound prepared from the mixture will be contaminated with a 5-methyl-2-(2-imidazolin-2-yl)nicotinic acid, ester or salt compound which has different herbicidal properties than the desired compound.

US 4,353,734 discloses maleic imide derivatives which are structurally comparable to compounds of formula III of the present invention wherein X is halogen and R₁ is a group -C(O)-R₂. However, no representative of compounds of formula III wherein X and R₁ are as defined above, is disclosed in US 4,353,734.

Helv. Chim Acta, 1988, 71: 486-492 (Adrian WALDNER) describes in 4. on page 491 of said publication a process which is analogous to the process according to the present invention. However, as starting materials, hydrazones are used (see formula 1 on page 487), whereas in the process according to the present invention 2-(alkoxymethyl)-2-propen-1-one derivates (see formula II; oxime derivatives with -C=N-OR₆) are used. Further, none of the products obtained has an alkoxymethyl substituent in 5-position of the pyridine moiety. The base used in this reference is triethyl amine.

It is, therefore, an object of the present invention to provide a process for the preparation of high purity 5-(alkoxymethyl)-2,3-pyridinedicarboximide compounds.

### SUMMARY OF THE INVENTION

The present invention provides an effective and efficient process for the preparation of a 5-(alkoxymethyl) -2,3-pyridinedicarboximide compound having the structural formula I 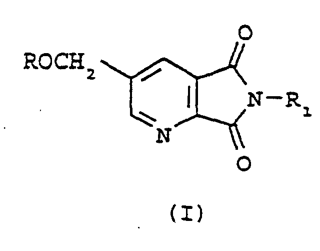 wherein
- R: is C₁-C₆alkyl;
- R₁: is hydrogen, C₁-C₆alkyl, C(O)R₂,
phenyl optionally substituted with any combination of from one to four halogen, C₁-C₄alkyl, C₁-C₄alkoxy, nitro or cyano groups,
benzyl optionally substituted on the phenyl ring with any combination of from one to four halogen, C₁-C₄alkyl, C₁-C₄alkoxy, nitro or cyano groups, or 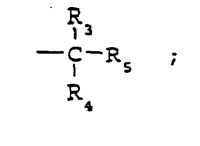
- R₂: is C₁-C₆alkyl,
phenyl optionally substituted with any combination of from one to four halogen, C₁-C₄alkyl, C₁-C₄alkoxy, nitro or cyano groups, or
benzyl optionally substituted on the phenyl ring with any combination of from one to four halogen, C₁-C₄alkyl, C₁-C₄alkoxy, nitro or cyano groups;
- R₃ and R₄: are each independently C₁-C₄alkyl; and
- R₅: is cyano or CONH₂,
which process comprises reacting an oxime of a 2-(alkoxymethyl)-2-propen-1-one compound having the structural formula II 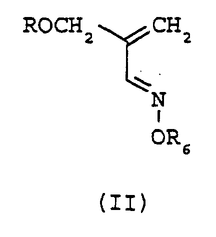 wherein R is as described above and R₆ is hydrogen or C₁-C₆alkyl with a substituted maleimide compound having the structural formula III 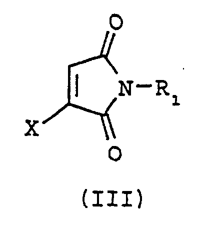 wherein R₁ is as described above and X is halogen, phenylsulfinyl or 1-imidazolyl and a base selected from the group consisting of a tri(C₂-C₄alkyl)amine, an alkali metal acetate and mixtures thereof in the presence of a solvent at an elevated temperature.

Surprisingly, it has been found that 5-(alkoxymethyl)-2,3-pyridinedicarboximide compounds are obtained in high purity when prepared by the effective and efficient process of the present invention.

In one preferred embodiment of the present invention, an oxime of a 2-(alkoxymethyl)-2-propen-1-one compound represented by formula II is reacted with at least about one molar equivalent of a substituted maleimide compound represented by formula III, and at least about one molar equivalent of a tri(C₂-C₄alkyl)amine or an alkali metal acetate or a mixture thereof, suitably at a temperature of at least 75 °C, e.g. a temperature of at least 90 °C, preferably in a temperature range of about 75 °C to 150 °C, more preferably about 90 °C to 135 °C, in the presence of a solvent having a boiling point of at least about 75 °C.

Advantageously, it has now been found that 5- (alkoxymethyl)-2,3-pyridinedicarboximide compounds are obtained in high purity by the effective and efficient process of the present invention. In contrast, 5-(alkoxymethyl)-2,3-pyridinedicarboximide compounds are contaminated with significant amounts of 5-methyl-2,3-pyridinedicarboximide compounds when prepared according to the procedure described in EP 308,084-A1.

The 5-(alkoxymethyl)-2,3-pyridinedicarboximide compounds may be isolated by diluting the reaction mixture with water and filtering the formula I product from the aqueous mixture. The product formula I compounds may also be isolated by concentrating the reaction mixture *in vacuo* and filtering the formula I product from the concentrated mixture. Alternatively, the reaction mixture may be integrated into the process used to prepare the final herbicidal agent without isolating the formula I compound.

Exemplary of halogen hereinabove are fluorine, chlorine, bromine and iodine.

The base is an especially important element of the present invention because it significantly reduces the production of 5-methyl-2,3-pyridinedicarboximide compounds. Tri(C₂-C₄alkyl)amines suitable for use in the process of the present invention include triethylamine, N,N-diethylisopropylamine, N,N-diisopropylethylamine and the like with triethylamine being preferred. Alkali metal acetates suitable for use in the process of this invention include sodium acetate, potassium acetate and the like with sodium acetate and potassium acetate being preferred. The base may suitably be used in an amount of at least one molar equivalent relative to the Formula I compound.

In another preferred embodiment of the present invention, a phase transfer catalyst is present. Preferably, the phase transfer catalyst is present when the alkali metal acetate is present. Phase transfer catalysts suitable for use in the present invention include any conventional phase transfer catalysts. Preferred phase transfer catalysts include crown ethers such as 18-crown-6 and 15-crown-5.

Solvents suitable for use in the process of the present invention preferably have a boiling point of at least about 75 °C and include aromatic hydrocarbons such as toluene, xylenes, mesitylene and mixtures thereof; halogenated aromatic hydrocarbons such as mono- and dihalobenzenes and mixtures thereof; polynuclear aromatic hydrocarbons such as naphthalene, alkylnaphthalenes and mixtures thereof; glycols such as 1,2-diethoxyethane and mixtures thereof; and mixtures thereof. Preferred solvents include toluene, xylenes, mesitylene, 1,2-diethoxyethane and mixtures thereof with toluene being more preferred.

In another embodiment of the present invention, an alkali metal carbonate such as sodium carbonate, potassium carbonate and the like is present. Preferably, the alkali metal carbonate is present when the tri(C₂-C₄alkyl)amine is present.

The process of the present invention is especially useful for the preparation of 5-(alkoxymethyl)-2,3-pyridinedicarboximide compounds wherein
R is methyl; and/or
R₁ is methyl, phenyl or 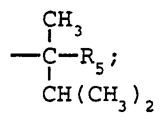 and/or
R₅ is cyano or CONH₂.

Oximes of 2-(alkoxymethyl)-2-propen-1-one compounds and substituted maleimide compounds which are particularly useful in the process of this invention are those wherein
R and R₆ are methyl; and/or
R₁ is methyl, phenyl or 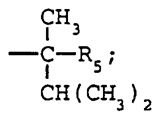 and/or
R₅ is cyano or CONH₂; and/or
X is Cl or Br.

The formula II oxime compounds of the present invention may be prepared by reacting a 2-alkoxymethylacrolein of formula IV with a substituted hydroxylamine of formula V optionally in the presence of a base. The reaction is shown below in Flow Diagram I.

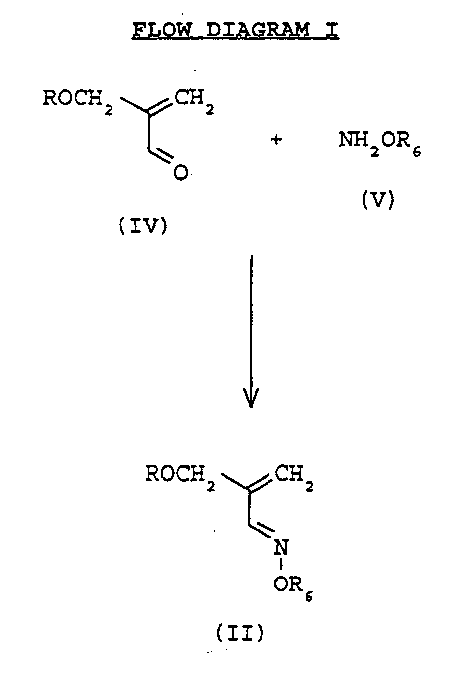

Alternatively, oxime compounds of formula II wherein R₆ is C₁-C₆alkyl may be prepared by reacting a formula II compound wherein R₆ is hydrogen with a dialkyl sulfate of formula VI and a base such as sodium hydroxide or an alkali metal alkoxide optionally in the presence of a phase transfer catalyst. The reaction scheme is shown in Flow Diagram II.

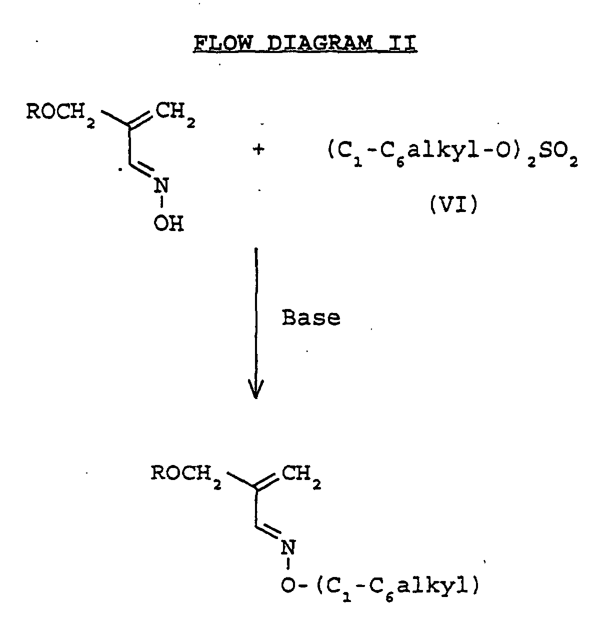

A method for the preparation of formula IV 2-alkoxymethylacroleins is described in U.S. 5,177,266. Formula III substituted maleimide compounds wherein X is halogen and R₁ is hydrogen, C₁-C₆alkyl, phenyl, substituted phenyl, benzyl, substituted benzyl or -CR₃R₄R₅ are known in the art and may be prepared according to the procedures described in EP 308,084-A1.

Substituted maleimide compounds wherein R₁ is C(O)R₂ may be prepared by reacting a formula III compound wherein R₁ is hydrogen with an acid chloride of formula VI in the presence of a solvent as shown in Flow Diagram III.

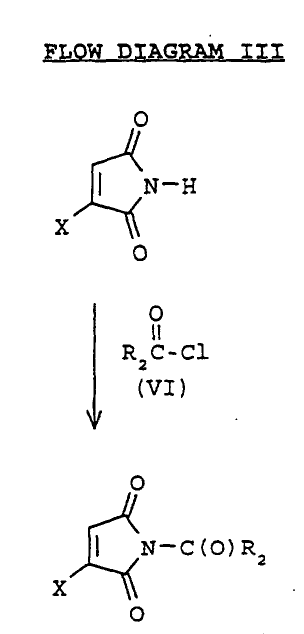

Formula III compounds wherein X is 1-imidazolyl may be prepared by reacting a formula III compound wherein X is Cl or Br with imidazole and a base such as a tri(C₁-C₄alkyl)amine in the presence of a solvent. And formula III compounds wherein X is phenylsulfinyl may be prepared by reacting a formula III compound wherein X is Cl or Br with thiophenol and a base such as an alkali metal acetate in the presence of a solvent to form an intermediate compound and oxidizing the intermediate compound with a conventional oxidizing agent in the presence of a solvent to form the desired formula III compound wherein X is phenylsulfinyl.

The present invention also provides a process for the preparation of a herbicidal 5-(alkoxymethyl)-2-(2-imidazolin-2-yl)-nicotinic acid, ester and salt compound having the formula 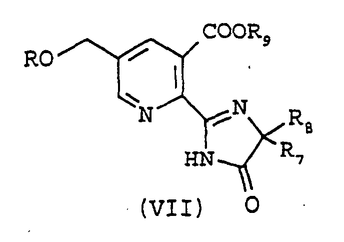 wherein
- R: is as defined above;
- R₇: is C₁-C₄ alkyl;
- R₈: is C₁-C₄ alkyl, C₃-C₆ cycloalkyl or R₇ and R₈ when taken together with the atom to which they are attached, represent a C₃-C₆ cycloalkyl group optionally substituted with methyl and
- R₉: is hydrogen, diloweralkylimino,
- C₁-C₁₂: alkyl optionally substituted with one of the following groups: C₁-C₃ alkoxy, halogen, hydroxy, C₃-C₆ cycloalkyl, benzyloxy, furyl, phenyl, halophenyl, lower alkylphenyl, lower alkoxyphenyl, nitrophenyl, carboxyl, loweralkoxycarbonyl, cyano or triloweralkylammonium;
- C₃-C₁₂: alkenyl optionally substituted with one of the following groups: C₁-C₃ alkoxy, phenyl, halogen or loweralkoxycarbonyl or with two C₁-C₃ alkoxy groups or two halogen groups;
- C₃-C₆: cycloalkyl optionally substituted with one or two C₁-C₃ alkyl groups;
or a cation preferably selected from the group consisting of alkali metals, alkaline earth metals, manganese, copper, iron, zinc, cobalt, lead, silver, nickel, ammonium and organic ammonium;
which process comprises:
(a) preparing a compound having the formula I 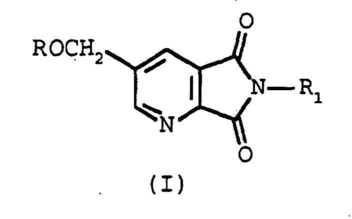 wherein R and R₁ are as defined above by a process as defined above; and
(b) converting the said compound having formula I into the compound having the formula VII.

The term "lower" as used above in relation to alkyl and alkoxy groups means that the alkyl or alkoxy group contains 1 to 6, preferably 1 to 4, carbon atoms. When used herein as a group or part of a group the term alkyl includes straight or branched chain alkyl groups such as methyl, ethyl, n-propyl, isopropyl, n-butyl, isobutyl, s-butyl and t-butyl.

The conversion of the compound having formula I into the compound having formula VII may be carried out in a variety of ways. One may plan routes by combining reactions known for the conversion of one carboxylic acid derivative into another.

Methods that may be used to create the imidazolinone herbicides are illustrated in the book "The Imidazolinone Herbicides" edited by D.L. Shaner and S.L. O'Connor, published 1991 by CRC Press, Boca Raton, Florida with particular reference to Chapter 2 entitled "Synthesis of the Imidazolinone Herbicides", pages 8-14 and the references cited therein. The following patent literature references also illustrate the methods that may be used to convert the carboxylic acid derivatives into imidazolinone final products:
U.S. Patent Nos. 5,371,229; 5,334,576; 5,250,694; 5,276,157; 5,110,930; 5,122,608; 5,206,368; 4,925,944; 4,921,961; 4,959,476; 5,103,009; 4,816,588; 4,748,244; 4,754,033; 4,757,146; 4,798,619; 4,766,218; 5,001,254; 5,021,078; 4,723,011; 4,709,036; 4,658,030; 4,608,079; 4,719,303; 4,562,257; 4,459,408; 4,459,409; 4,460,776; 4,125,727 and 4,758,667, and European Patent Application Nos. EP-A-0-041,623 and EP-A-0-308,084.

In order to facilitate a further understanding of the invention, the following examples are presented primarily for the purpose of illustrating more specific details thereof. The invention should not be deemed limited by the examples as the full scope of the invention is defined in the claims.

### EXAMPLE 1

### Preparation of 5-(Methoxymethyl)-N-phenyl-2,3-pyridinedicarboximide

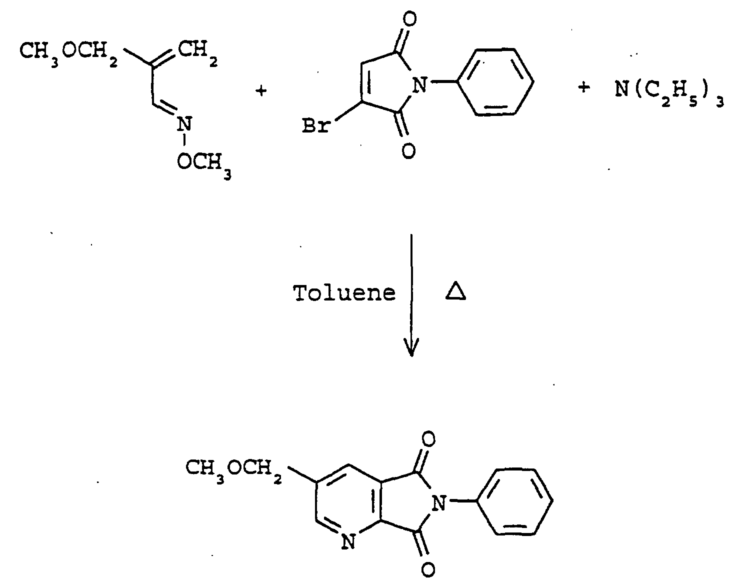

A solution of the O-methyloxime of 2-(methoxymethyl)-2-propen-1-one (9.0 g, 96% pure, 67 mmol) in toluene (112 g) is heated at 97 °C for 27 hours. During the heating period, 2-bromo-N-phenylmaleimide (27.75 g, 110 mmol) and triethylamine (17.9 g, 177 mmol) are added portionwise to the reaction mixture. The final reaction mixture is filtered to remove solids, washed with water and concentrated *in vacuo* to give the title product as an orange solid (10.9 g, 61% yield) having a 5-(methoxymethyl)-N-phenyl-2,3-pyridinedicarboximide to 5-methyl-N-phenyl-2,3-pyridinedicarboximide ratio of 233:1.

### EXAMPLES 2-9

The procedure of Example 1 is repeated using a variety of conditions and the results are summarized in Table I.

As can be seen from the data in Examples 1-9, the process of the present invention (Examples 1-7) produces 5- (methoxymethyl) -N-phenyl-2,3-pyridinedicarboximide which is contaminated with a significantly smaller amount of 5-methyl-N-phenyl-2,3-pyridinedicarboximide than the process described in EP 308,084-A1 (Examples 8 and 9).

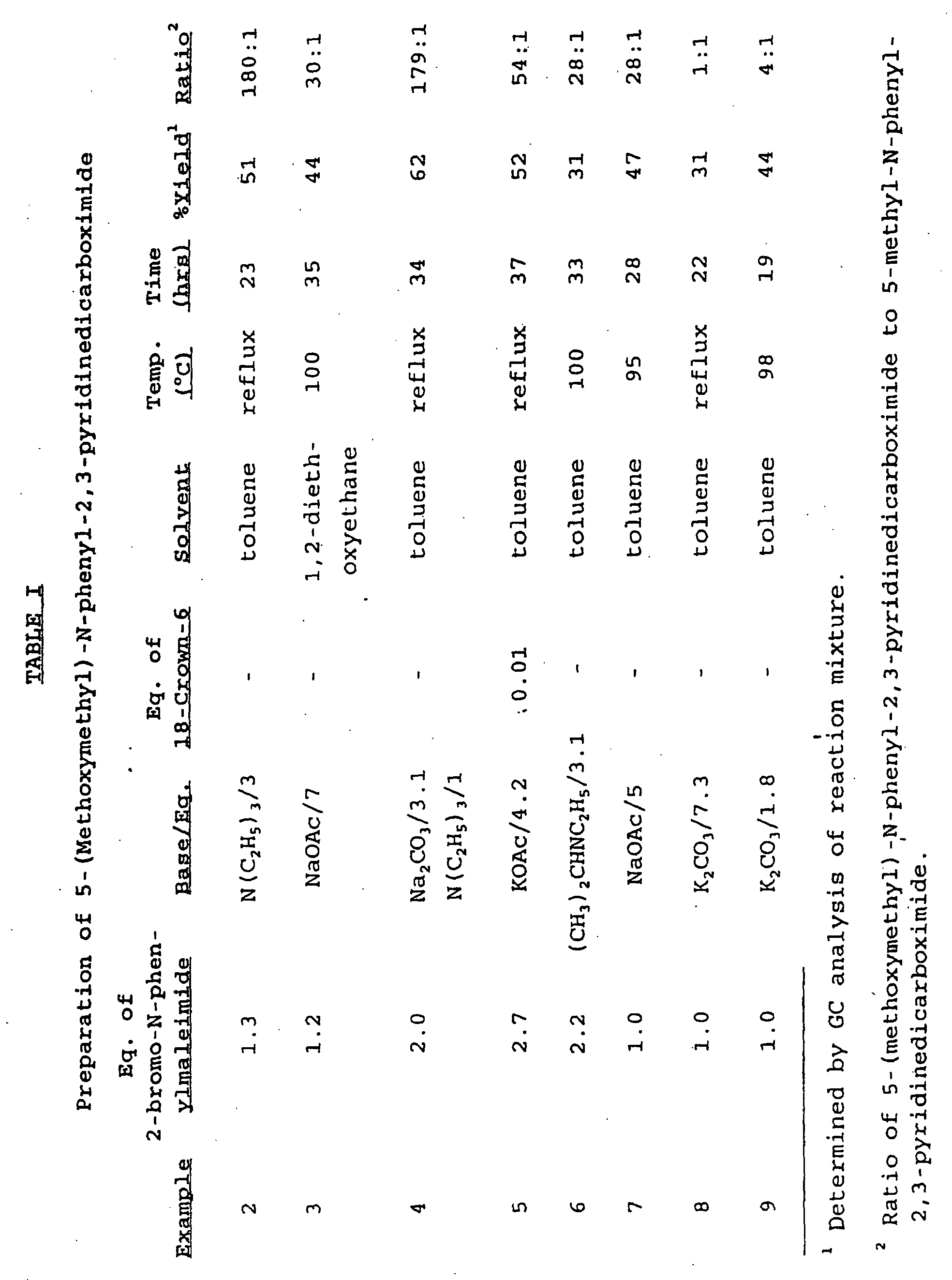

### EXAMPLES 10-12

Using essentially the same procedure as described in Example 1, but substituting 2-chloro-N-phenylmaleimide for 2-bromo-N-phenylmaleimide, 5-methoxymethyl-N-phenyl-2,3-pyridinedicarboximide is produced in the yields shown in Table II.

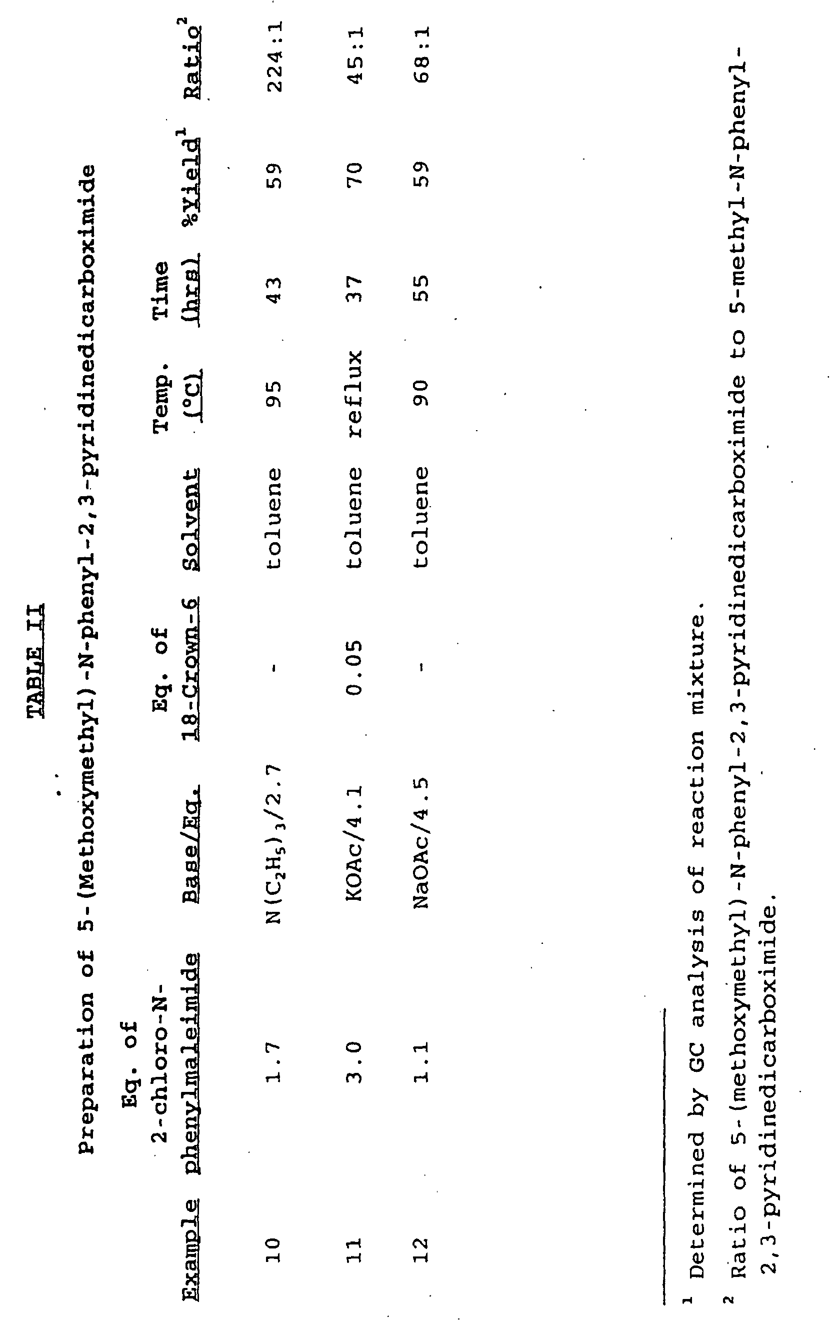

### EXAMPLE 13

### Preparation of 5-(Methoxymethyl)-N-methyl-2,3-pyridinedicarboximide

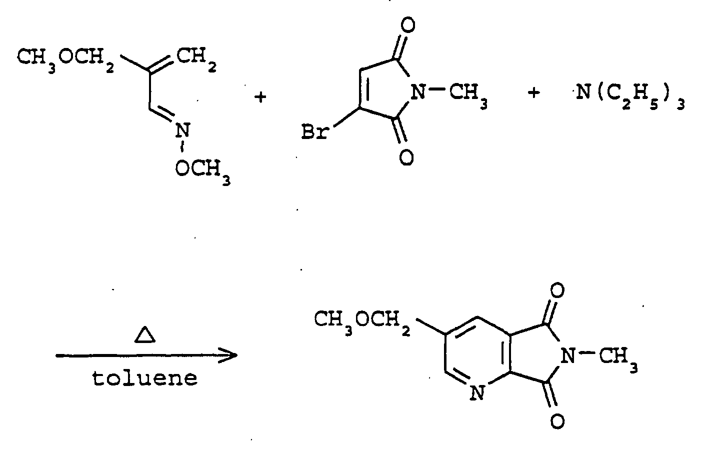

A solution of O-methyloxime of 2-(methoxymethyl)-2-propen-1-one (9.9 g, 84% pure, 64 mmol) in toluene (112 g) is heated at 100 °C for 44 hours. During the heating period, 2-bromo-N-methylmaleimide (20.35 g, 107 mmol) and triethylamine (16.9 g, 167 mmol) are added portionwise to the reaction mixture. The final reaction mixture is filtered to remove solids, washed with water and concentrated *in vacuo* to give the title product as an orange solid (8.1 g, 61% yield) having a 5-(methoxymethyl)-N-methyl-2,3-pyridinedicarboximide to 5-methyl-N-methyl-2,3-pyridinedicarboximide ratio of 316:1.

### EXAMPLES 14 and 15

Using essentially the same procedure as described in Example 13, 5-(methoxymethyl)-N-methyl-2,3-pyridinedicarboximide is produced in the yields shown in Table III.

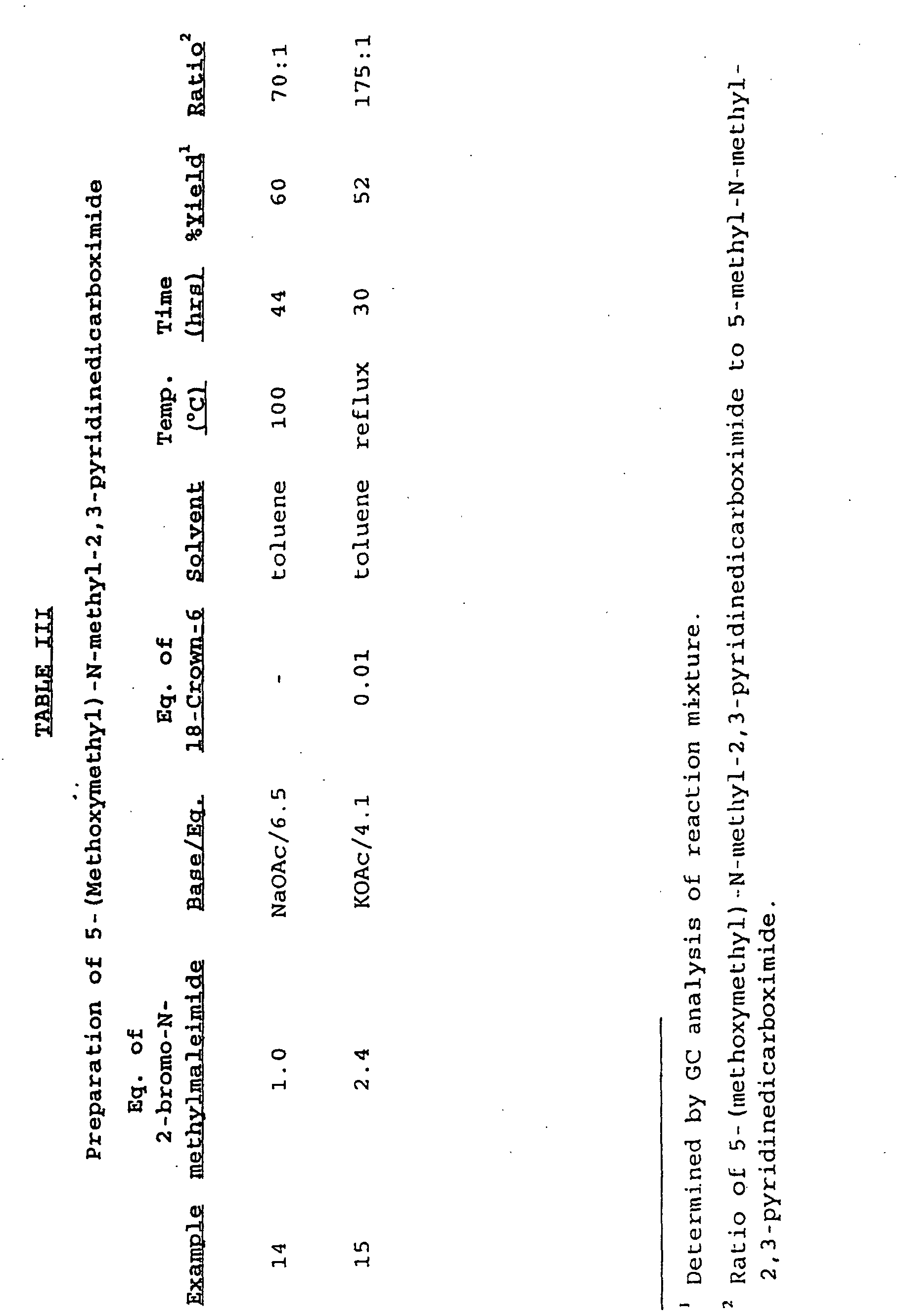

### EXAMPLES 16-18

Using essentially the same procedure as described in Example 13, but substituting 2-chloro-N-methylmaleimide for 2-bromo-N-methylmaleimide, 5-(methoxymethyl)-N-methyl-2,3-pyridinedicarboximide is produced in the yields shown in Table IV.

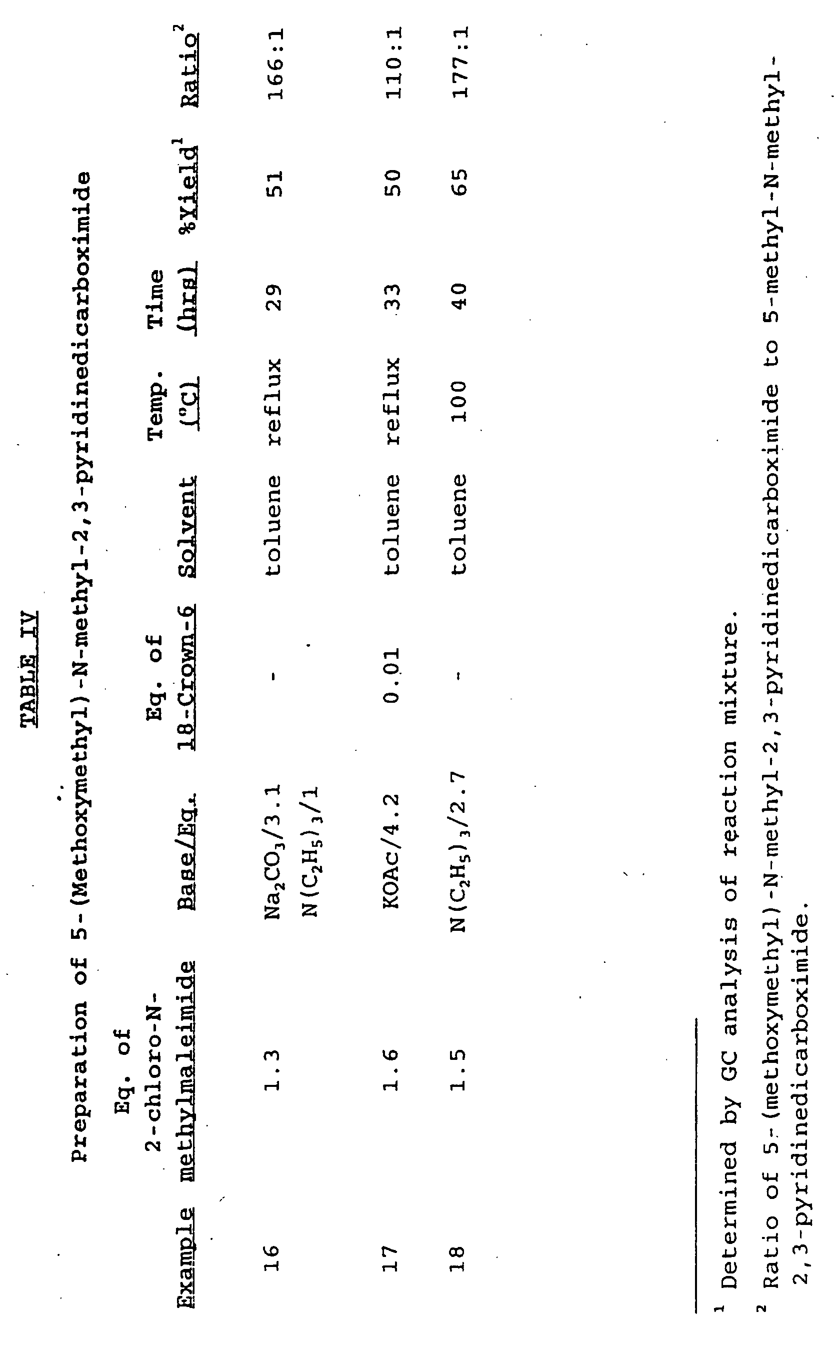

### EXAMPLE 19

### Preparation of 5,7-Dihydro-α-isopropyl-3-(methoxymethyl)-α-methyl-5,7-dioxo-6H-pyrrolo [3,4-b]pyridine-6-acetonitrile

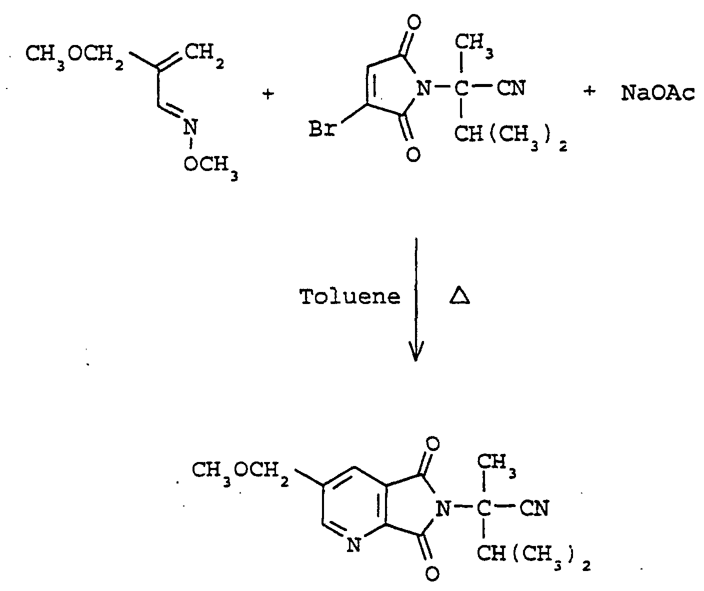

A mixture of 3-bromo-2,5-dihydro-α-isopropyl-α-methyl-2,5-dioxopyrrole-1-acetonitrile (0.6 g, 90% pure, 1.99 mmol) in toluene (12 g) is heated at 95 °C for 41 hours. During the heating period, the O-methyloxime of 2-(methoxymethyl)-2-propen-1-one (0.27 g, 2.0 mmol) and sodium acetate (0.76 g, 9.2 mmol) are added portionwise to the reaction mixture. The final reaction mixture is filtered to remove solids, washed with water and concentrated in vacuo to give the title product as a red oil (0.40 g, 70% yield) having a 5,7-dihydro-α-isopropyl-3-(methoxymethyl)-α-methyl-5,7-dioxo-6H-pyrrolo[3,4-b]pyridine-6-acetonitrile to 5,7-dihydro-α-isopropyl-3,α-dimethyl-5,7-dioxo-6H-pyrrolo [3,4-b]pyridine-6-acetonitrile ratio of 61:1.

Using essentially the same procedure, but in the absence of a base, 5,7-dihydro-α-isopropyl-3-(methoxymethyl)-α-methyl-5,7-dioxo-6H-pyrrolo[3,4-b]pyridine-6-acetonitrile is obtained in 32% yield with a 5,7-dihydro-α-isopropyl-3-(methoxymethyl)-α-methyl-5,7-dioxo-6H-pyrrolo[3,4-b]pyridine-6-acetonitrile to 5,7-dihydro-α-isopropyl-3,α-dimethyl-5,7-dioxo-6H-pyrrolo[3,4-b]pyridine-6-acetonitrile ratio of 2:1.

### EXAMPLE 20

### Preparation of the Oxime of 2-(methoxymethyl)-2-propen-1-one

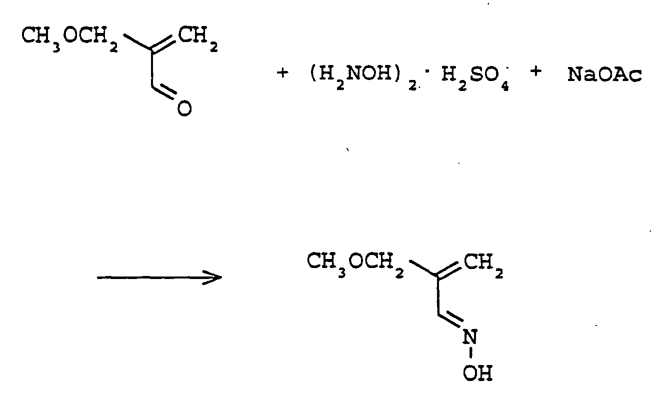

A mixture of hydroxylamine sulfate (3.81 g, 23.2 mmol) and sodium acetate (3.52 g, 4.29 mmol) in water (30 g) is treated dropwise with 2-methoxymethylacrolein (5.1 g, 70% pure) over 15 minutes, stirred at room temperature for 5 hours and extracted with methylene chloride. The organic extract is dried over anhydrous magnesium sulfate and concentrated *in vacuo* to give the title product as a yellow oil (3.3 g, 80% yield).

### EXAMPLE 21

### Preparation of the O-methyloxime of 2-(methoxymethyl)-2-propen-1-one

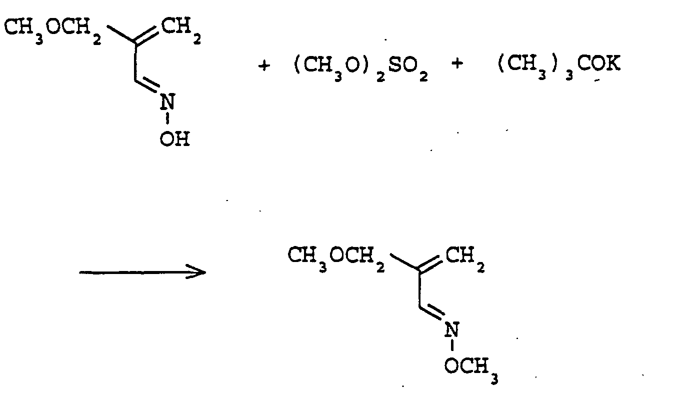

A mixture of the oxime of 2-(methoxymethyl)-2-propen-1-one (1.8 g, 10.4 mmol, 67% pure) and potassium tert-butoxide (1.93 g, 17.2 mmol) in tetrahydrofuran (40 g) is stirred at 5 ° to 10 °C for 10 minutes, treated dropwise with dimethyl sulfate (2.37 g, 18.8 mmol), stirred for 1.2 hours, filtered to remove solids and concentrate *in vacuo* to obtain a residue. The residue is dissolved in methylene chloride and the resultant solution is washed sequentially with water and brine, dried over anhydrous magnesium sulfate and concentrated *in vacuo* to give the title product as a yellow oil (1.4 g, 74% yield).

### EXAMPLE 22

### Preparation of the O-methyloxime of 2-(methoxymethyl)-2-propen-1-one

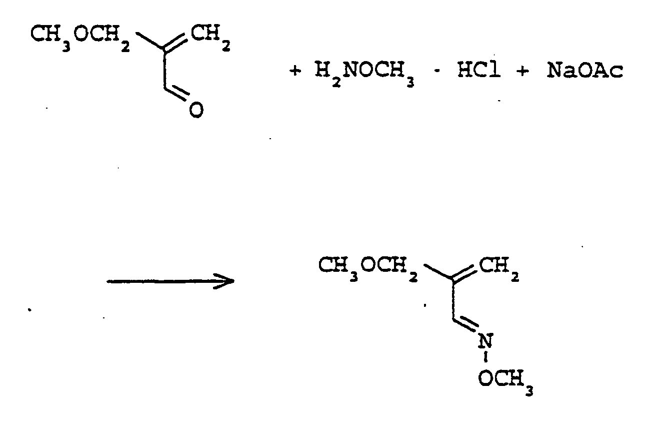

A mixture of methoxylamine hydrochloride (21.0 g, 0.25 mol) and sodium acetate (22.4 g, 0.27 mol) in water (159 g) is treated dropwise with 2-methoxymethylacrolein (21.0 g, 90% pure, 0.19 mol) over ten minutes at 21° to 29°C, stirred overnight at room temperature and extracted with methylene chloride. The organic extract is washed sequentially with water and brine, dried over anhydrous magnesium sulfate and concentrated *in vacuo* to give the title product as a yellow oil (26.1 g). A portion of the yellow oil is distilled under reduced pressure (50° to 70°C, 16 hPa (16 mm Hg)) to give the title product as a clear, colorless oil.

## Claims

1. A process for the preparation of a 5-(alkoxymethyl)-2,3-pyridinedicarboximide compound having the structural formula I 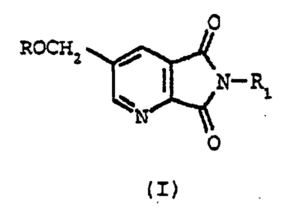 wherein
R is C₁-C₆alkyl;
F₁ is hydrogen, C₁-C₆alkyl, C(O)R₂,
phenyl optionally substituted with any combination of from one to four halogen, C₁-C₄alkyl, C₁-C₄alkoxy, nitro or cyano groups,
benzyl optionally substituted on the phenyl ring with any combination of from one to four halogen, C₁-C₄alkyl, C₁-C₄alkoxy, nitro or cyano groups, or 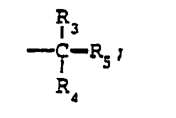
R₂ is C₁-C₆alkyl,
phenyl optionally substituted with any combination of from one to four halogen, C₁-C₄alkyl, C₁-C₄alkoxy, nitro or cyano groups, or
benzyl optionally substituted on the phenyl ring with any combination of from one to four halogen, C₁-C₄alkyl, C₁-C₄alkoxy, nitro or cyano groups;
R₃ and R₄ are each independently C₁-C₄alkyl; and
R₅ is cyano or CONH₂,
which process comprises reacting an oxime of a 2-(alkoxymethyl)-2-propen-1-one compound having the structural formula II 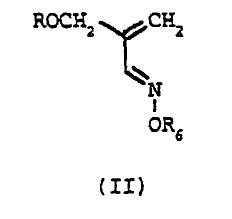 wherein R is as described above and R₆is hydrogen or C₁-C₄alkyl with a substituted maleimide compound having the structural formula III 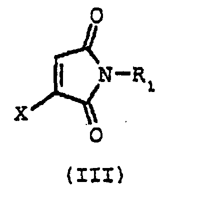 wherein R₁ is as described above and X is halogen, phenylsulfinyl or 1-imidazolyl and a base selected from the group consisting of a tri(C₂-C₄alkyl)amine, an alkali metal acetate and mixtures thereof in the presence of a solvent at an elevated temperature.

2. The process according to claim 1 wherein the base is selected from the group consisting of triethylamine, sodium acetate and potassium acetate and is present in an amount of at least one molar equivalent relative to the formula I compound.

3. The process according to claim 1 or claim 2 further comprising a phase transfer catalyst selected from 18-crown-6 or 15-crown-5.

4. The process according to any one of claims 1 to 3 further comprising an alkali metal carbonate selected from sodium carbonate and potassium carbonate,

5. The process according to any one of claims 1 to 4 wherein R and R₆ are methyl and/or
R₁ is methyl, phenyl or 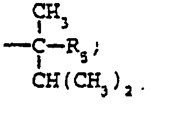 and/or
X is Cl or Br.

6. The process according to any one of claims 1 to 5 wherein the solvent is selected from the group consisting of an aromatic hydrocarbon, a halogenated aromatic hydrocarbon, a polynuclear aromatic hydrocarbon, a glycol and mixtures thereof, and the boiling point of the solvent is at least 75 °C.

7. The process according to any one of claims 1 to 6 wherein the formula II compound is reacted with the formula III compound and the base at a temperature of above 75 °C.

8. A process for the preparation of a herbicidal imidazolinone compound having the formula VII 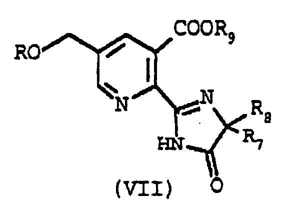 wherein
R is as defined in Claim 1;
R₇ is C₁-C₄ alkyl;
R₈ is C₁-C₄ alkyl, C₃-C₆ cycloalkyl or R₇ and R₈ when taken together with the atom to which they are attached, represent a C₃-C₆ cycloalkyl group optionally substituted with methyl and
R₉ is hydrogen, di-C₁-C₆-alkylimino,
C₁-C₁₂ alkyl optionally substituted with one of the following groups: C₁-C₃ alkoxy, halogen, hydroxy, C₃-C₆ cycloalkyl, benzyloxy, furyl, phenyl, halophenyl, lower alkylphenyl, lower alkoxyphenyl, nitrophenyl, carboxyl, loweralkoxycarbonyl, cyano or triloweralkylammonium;
C₃-C₁₂ alkenyl optionally substituted with one of the following groups: C₁-C₃ alkoxy, phenyl, halogen or loweralkoxycarbonyl or with two C₁-C₃ alkoxy groups or two halogen groups;
C₃-C₆ cycloalkyl optionally substituted with one or two C₁-C₃ alkyl groups;
or a cation;
which process comprises:
(a) preparing a compound having the formula I 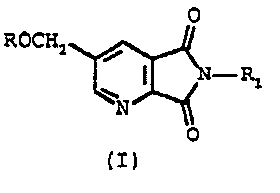 wherein R and R₁ are as defined in Claim 1 by a process as claimed in Claim 1; and
(b) converting the compound having formula I into the compound having the formula VII.

## Patentansprüche

1. Verfahren zur Herstellung einer 5-(Alkoxymethyl)-2,3-pyridindicarboximidverbindung mit der Strukturformel I 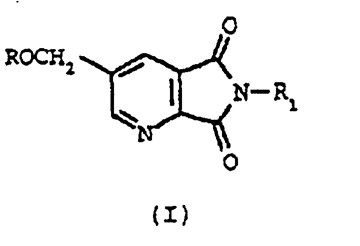 in welcher
R für C₁-C₆-Alkyl steht;
R₁ für Wasserstoff, C₁-C₆-Alkyl, C(O)R₂,
Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist,
Benzyl, welches gegebenenfalls am Phenylring durch eine beliebige Kombination von ein bis vier Halogen-, C₁-C₄-Alkyl-, C₁-C₄-Alkoxy-, Nitro- oder Cyanogruppen substituiert ist, oder 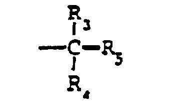 steht;
R₂ für C₁-C₆-Alkyl,
Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, oder
Benzyl, welches gegebenenfalls am Phenylring durch eine beliebige Kombination von ein bis vier Halogen-, C₁-C₄-Alkyl-, C₁-C₄-Alkoxy-, Nitro- oder Cyanogruppen substituiert ist, steht;
R₃ und R₄ jeweils unabhängig voneinander für C₁-C₄-Alkyl stehen; und
R₅ für Cyano oder CONH₂ steht,
bei dem man ein Oxim einer 2-(Alkoxymethyl)-2-propen-1-onverbindung mit der Strukturformel II 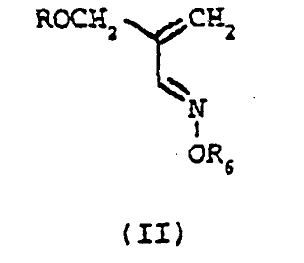 in welcher R wie oben beschrieben ist und
R₆ für Wasserstoff oder C₁-C₆-Alkyl steht,
mit einer substituierten Maleinimidverbindung mit der Strukturformel III 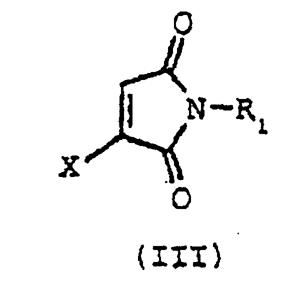 in welcher R₁ wie oben beschrieben ist und
X für Halogen, Phenylsulfinyl oder 1-Imidazolyl steht, und einer aus einem Tri(C₂-C₄-alkyl)amin, einem Alkaliacetat und Mischungen davon ausgewählten Base in Gegenwart eines Lösungsmittels bei erhöhter Temperatur umsetzt.

2. Verfahren nach Anspruch 1, wobei die Base aus Triethylamin, Natriumacetat und Kaliumacetat ausgewählt ist und in einer Menge von wenigstens einem Moläquivalent, bezogen auf die Verbindung der Formel I, vorliegt.

3. Verfahren nach Anspruch 1 oder 2, weiterhin umfassend einen aus 18-Krone-6 und 15-Krone-5 ausgewählten Phasentransferkatalysator.

4. Verfahren nach einem der Ansprüche 1 bis 3, weiterhin umfassend ein aus Natriumcarbonat und Kaliumcarbonat ausgewähltes Alkalicarbonat.

5. Verfahren nach einem der Ansprüche 1 bis 4, wobei R und R₆ für Methyl stehen und/oder R₁ für Methyl, Phenyl oder. 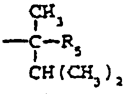 steht;
und/oder X für Cl oder Br steht.

6. Verfahren nach einem der Ansprüche 1 bis 5, wobei das Lösungsmittel aus der aus einem aromatischen Kohlenwasserstoff, einem aromatischen Halogenkohlenwasserstoff, einem mehrkernigen aromatischen Kohlenwasserstoff, einem Glycol und Mischungen davon bestehenden Gruppe ausgewählt ist und der Siedepunkt des Lösungsmittels wenigstens 75°C beträgt.

7. Verfahren nach einem der Ansprüche 1 bis 6, wobei man die Verbindung der Formel II bei einer Temperatur von über 75°C mit der Verbindung der Formel III und der Base umsetzt.

8. Verfahren zur Herstellung einer herbiziden Imidazolinonverbindung mit der Formel VII 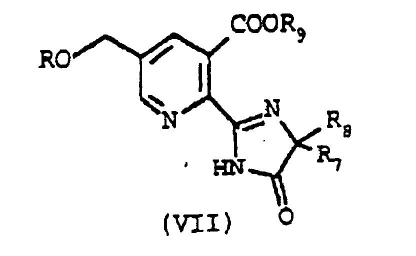 wobei
R wie in Anspruch 1 definiert ist;
R₇ für C₁-C₄-Alkyl steht;
R₈ für C₁-C₄-Alkyl oder C₃-C₆-Cycloalkyl steht oder R₇ und R₈ zusammen mit dem Atom, an das sie gebunden sind, für eine gegebenenfalls Methyl-substituierte C₃-C₆-Cycloalkylgruppe stehen, und
R₉ für Wasserstoff, Di-C₁-C₄-alkylimino, C₁-C₁₂-Alkyl, welches gegebenenfalls durch eine der folgenden Gruppen substituiert ist: C₁-C₃-Alkoxy, Halogen, Hydroxyl, C₃-C₆-Cycloalkyl, Benzyloxy, Furyl, Phenyl, Halogenphenyl, niederes Alkylphenyl, niederes Alkoxyphenyl, Nitrophenyl, Carboxyl, niederes Alkoxycarbonyl, Cyano oder Triniederes Alkylammonium;
C₃-C₁₂-Alkenyl, welches gegebenenfalls durch eine der folgenden Gruppen substituiert ist: C₁-C₃-Alkoxy, Phenyl, Halogen oder niederes Alkoxycarbonyl oder durch zwei C₁-C₃-Alkoxygruppen oder zwei Halogengruppen;
C₃-C₆-Cycloalkyl, welches gegebenenfalls durch ein oder zwei C₁-C₃-Alkylgruppen substituiert ist;
oder ein Kation steht,
bei dem man:
(a) eine Verbindung mit der Formel I 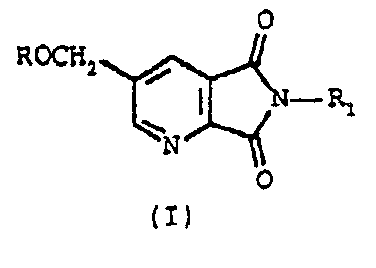 in welcher R und R₁ wie in Anspruch 1 definiert sind, durch ein Verfahren nach Anspruch 1 darstellt; und
(b) die Verbindung mit der Formel I in die Verbindung mit der Formel VII umwandelt.

## Revendications

1. Procédé pour la préparation d'un 5-(alcoxyméthyl)-2,3-pyridinedicarboximide ayant la formule développée I 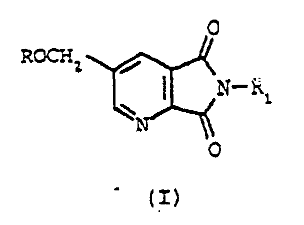 dans laquelle
R est un groupe alkyle en C₁-C₆ ;
R₁ est l'hydrogène, un groupe alkyle en C₁-C₆, C(O)R₂,
phényle facultativement substitué par toute combinaison d'un à quatre groupes halogéno, alkyle en C₁-C₄, alcoxy en C₁-C₄, nitro ou cyano,
benzyle facultativement substitué sur le noyau phényle par toute combinaison d'un à quatre groupes halogéno, alkyle en C₁-C₄, alcoxy en C₁-C₄, nitro ou cyano, ou 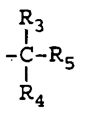
R₂ est un groupe alkyle en C₁-C₆,
phényle facultativement substitué par toute combinaison d'un à quatre groupes halogéno, alkyle en C₁-C₄, alcoxy en C₁-C₄, nitro ou cyano, ou
benzyle facultativement substitué sur le noyau phényle par toute combinaison d'un à quatre groupes halogéno, alkyle en C₁-C₄, alcoxy en C₁-C₄, nitro ou cyano ;
R₃ et R₄ sont chacun indépendamment un groupe alkyle en C₁-C₄ ; et
R₅ est un groupe cyano ou CONH₂,
lequel procédé comprend la réaction d'une oxime d'une 2- (alcoxyméthyl) -2-propène-1-one ayant la formule développée II 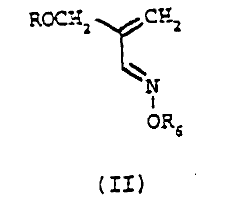 dans laquelle R est tel que défini ci-dessus et R₆ est l'hydrogène ou un groupe alkyle en C₁-C₄, avec un maléimide substitué ayant la formule développée III 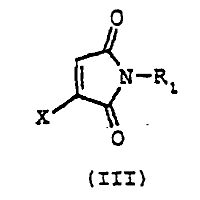 dans laquelle R₁ est tel que défini ci-dessus et X est un groupe halogéno, phénylsulfinyle ou 1-imidazolyle et une base choisie dans la classe formée par une tri(alkyle en C₂-C₄)amine, un acétate de métal alcalin et leurs mélanges, en présence d'un solvant à une température élevée.

2. Procédé selon la revendication 1, dans lequel la base est choisie dans la classe formée par la triéthylamine, l'acétate de sodium et l'acétate de potassium et est présente en une quantité d'au moins un équivalent molaire par rapport au composé de formule I.

3. Procédé selon la revendication 1 ou la revendication 2, comprenant de plus un catalyseur de transfert entre phases choisi parmi le 18-couronne-6 et le 15-couronne-5.

4. Procédé selon l'une quelconque des revendications 1 à 3, comprenant de plus un carbonate de métal alcalin choisi parmi le carbonate de sodium et le carbonate de potassium.

5. Procédé selon l'une quelconque des revendications 1 à 4, dans lequel R et R₆ sont des groupes méthyle et/ou R₁ est un groupe méthyle, phényle ou 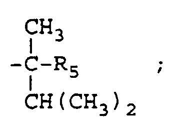 et/ou
X est Cl ou Br.

6. Procédé selon l'une quelconque des revendications 1 à 5, dans lequel le solvant est choisi dans la classe formée par un hydrocarbure aromatique, un hydrocarbure aromatique halogéné, un hydrocarbure aromatique polycyclique, un glycol et leurs mélanges, et le point d'ébullition du solvant est d'au moins 75°C.

7. Procédé selon l'une quelconque des revendications 1 à 6, dans lequel le composé de formule II est amené à réagir avec le composé de formule III et la base à une température supérieure à 75°C.

8. Procédé pour la préparation d'une imidazolinone herbicide ayant la formule VII 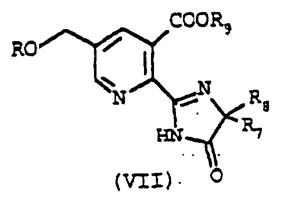 dans laquelle
R est tel que défini dans la revendication 1 ;
R₇ est un groupe alkyle en C₁-C₄ ;
R₈ est un groupe alkyle en C₁-C₄, cycloalkyle en C₃-C₆, où bien R₇ et R₈, lorsqu'ils sont pris ensemble avec l'atome auquel ils sont attachés, représentent un groupe cycloalkyle en C₃-C₆ facultativement substitué par un groupe méthyle, et
R₉ est l'hydrogène, un groupe di(alkyle en C₁-C₆)imino,
alkyle en C₁-C₁₂ facultativement substitué par l'un des groupes suivants : alcoxy en C₁-C₃, halogéno, hydroxyle, cycloalkyle en C₃-C₆, benzyloxy, furyle, phényle, halogénophényle, (alkyle inférieur)phényle, (alcoxy inférieur)phényle, nitrophényle, carboxyle, (alcoxy inférieur)carbonyle, cyano ou tri(alkyle inférieur)ammonium ;
alcényle en C₃-C₁₂ facultativement substitué par l'un des groupes suivants : alcoxy en C₁-C₃, phényle, halogéno ou (alcoxy inférieur)carbonyle, ou par deux groupes alcoxy en C₁-C₃ ou deux groupes halogéno ;
cycloalkyle en C₃-C₆ facultativement substitué par un ou deux groupes alkyle en C₁-C₃ ;
ou un cation ;
lequel procédé comprend :
(a) la préparation d'un composé ayant la formule I 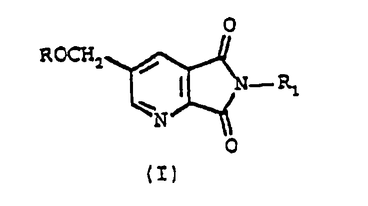 dans laquelle R et R₁ sont tels que définis dans la revendication 1, par un procédé tel que revendiqué dans la revendication 1 ; et
(b) la conversion du composé ayant la formule I en le composé ayant la formule VII.
